# EUROPEAN PATENT APPLICATION

(11) **EP 4 461 304 A1**
(43) Date of publication of application: **13.11.2024**
(21) Application number: 23752945.8
(22) Date of filing: 09.02.2023
(51) Int. Cl.: A61K 38/08, A23L 33/175, A61P 1/16, A61P 29/00, A61P 31/14, A61P 31/20

(54) **COMPOSITION FOR PREVENTING OR IMPROVING HEPATIC DYSFUNCTION**

(30) Priority: 10.02.2022 JP 2022019657
(71) Applicant: Kyowa Hakko Bio Co., Ltd., Tokyo 100-8185 (JP); KIRIN HOLDINGS KABUSHIKI KAISHA, Nakano-ku, Tokyo 164-0001 (JP)
(72) Inventor: MORITA Masahiko, Tokyo 100-8185 (JP); SAKO Naoki, Tokyo 1640001 (JP)
(74) Representative: Vossius & Partner Patentanwälte Rechtsanwälte mbB
(86) International application number: PCT/JP2023/004432
(87) International publication number: WO 2023/153483

(57) **Abstract**

This composition for preventing or improving hepatic dysfunction contains glutathione trisulfide or a pharmacologically acceptable salt thereof.

## Description

### Technical Field

The present invention relates to a composition for preventing or improving hepatic dysfunction and more specifically, relates to a composition for preventing or improving hepatic dysfunction, which comprises glutathione trisulfide or a pharmacologically acceptable salt thereof. The present invention also relates to a composition for preventing or improving symptoms related to hepatic dysfunction.

### Background Art

The liver is a reddish-brown organ having the largest volume and weight among all organs, and it weighs 1,000 to 1,500 g in adult males and 900 to 1,300 g in adult females. The liver contributes about 40% of the basal metabolism in all organs and plays a wide range of important roles, including respiration, maintenance of body temperature, metabolism of nutrients such as carbohydrates, lipids, proteins, and vitamins, and detoxification of nitrogen metabolites generated by harmful xenobiotics or biological activities.

The reduction of hepatic function may be caused by hepatic dysfunction or various hepatic function diseases associated with hepatic dysfunction. In association with the progression thereof, subjective symptoms such as fatigue, malaise, nausea, anorexia, fever, and the like are observed, and it is also known that these diseases become a cause of complications associated with the diseases. Examples of hepatic function diseases include hepatitis B, hepatitis C, alcoholic liver disorder, or nonalcoholic fatty liver disease, and these hepatic function diseases have become one of the most frequent lifestyle diseases of modern people, in association with an increase in lifestyle-related diseases based on obesity, diabetes mellitus, dyslipidemia, and the like (Non-Patent Literature 1).

It is important to protect the liver at an early stage and to prevent or improve hepatic dysfunction appropriately and reliably since there is a risk that hepatic dysfunction will develop into serious diseases such as hepatic cirrhosis and liver cancer in a case where it is left unattended.

One of the substances known to have a protective effect on the liver is reduced glutathione (GSH) (Non-Patent Literature 2 and 3). On the other hand, the effect on liver function obtained by administering glutathione trisulfide (GSSSG) and the like which can be converted in vivo into oxidized glutathione (GSSG), glutathione polysulfide, and the like, was completely unknown until now.

Incidentally, it has been reported that there is a possibility that polysulfides have a physiological function such as oxidative stress protection through the control of the oxidation/reduction state of cells (for example, Non-Patent Literature 4 and 5). As a production method for such a trisulfide compound, for example, the method described in Patent Literature 1 is known.

### Citation List

### Patent Literature

[Patent Literature 1] WO2018/117186

### [Non-Patent Literature]

[Non-Patent Literature 1] T. Okanoue, et al., "Liver function tests and liver injury - focusing on AST/ALT and PLT count", Health Evaluation and Promotion, 42(2), 307-312, (2015)
[Non-Patent Literature 2] Y. Sugimura, et al., "Effect of Orally Administered Reduced- and Oxidized-Glutathione against Acetaminophen-Induced Liver Injury in Rats", Journal of Nutritional Science and Vitaminology, 44, 613-624, (1998)
[Non-Patent Literature 3] Y. Honda, et al., "Efficacy of glutathione for the treatment of nonalcoholic fatty liver disease: an open-label, single-arm, multicenter, pilot study", BMC Gastroenterology, 17:96, (2017)
[Non-Patent Literature 4] T. Ida, et al., "Reactive cysteine persulfides and S-polythiolation regulate oxidative stress and redox signaling", Proceedings of the National Academy of Sciences of the United States of America, 111(21), 7606-7611, (2014)
[Non-Patent Literature 5] Kunikata et al., "Metabolomic profiling of reactive persulfides and polysulfides in the aqueous and vitreous humors", Scientific Reports, 7, 41984 (2017)

### Summary of Invention

### Technical Problem

An object of the present invention is to provide a composition for preventing or improving hepatic dysfunction.

### Solution to Problem

The inventors of the present invention found that AST and ALT levels in the blood plasma decrease in a case where liver disorder is induced in mice to which glutathione trisulfide have been administered, whereby the present invention was completed. The present invention relates to [1] to [11], [3'], and [10'] described below.
[1] A composition for preventing or improving hepatic dysfunction, comprising glutathione trisulfide or a pharmacologically acceptable salt thereof.
[2] The composition according to [1], wherein the hepatic dysfunction is a condition that is not accompanied with a hepatic function disease, where abnormal liver function is observed, or a condition that is accompanied with a hepatic function disease, where abnormal liver function is observed.
[3] The composition according to [2], wherein the hepatic function disease is at least one selected from the group consisting of hepatitis B, hepatitis C, alcoholic liver disorder, nonalcoholic fatty liver disease, and chronic hepatitis.
   The composition according to [1] or [2], wherein the hepatic dysfunction is hepatic dysfunction due to reactive oxygen disorder.
[4] The composition according to any one of [1] to [3] and [3'], wherein the composition is a food composition.
[5] The composition according to any one of [1] to [3] and [3'], wherein the composition is a pharmaceutical composition.
[6] A method for preventing or improving hepatic dysfunction, comprising administering glutathione trisulfide or a pharmacologically acceptable salt thereof to a subject in need thereof.
[7] Glutathione trisulfide or a pharmacologically acceptable salt thereof for use in preventing or improving hepatic dysfunction.
[8] Use of glutathione trisulfide or a pharmacologically acceptable salt thereof for the manufacture of a composition for preventing or improving hepatic dysfunction.
[9] The method, the glutathione trisulfide or the pharmacologically acceptable salt thereof for use, or the use according to any one of [6] to [8], wherein the hepatic dysfunction is a condition that is not accompanied with a hepatic function disease, where abnormal liver function is observed, or a condition that is accompanied with a hepatic function disease, where abnormal liver function is observed.
[10] The method, the glutathione trisulfide or the pharmacologically acceptable salt thereof for use, or the use according to [9], wherein the hepatic function disease is at least one selected from the group consisting of hepatitis B, hepatitis C, alcoholic liver disorder, nonalcoholic fatty liver disease, and chronic hepatitis.
   The method, the glutathione trisulfide or the pharmacologically acceptable salt thereof for use, or the use according to any one of [6] to [9], wherein the hepatic dysfunction is hepatic dysfunction due to reactive oxygen disorder.
[11] A composition for preventing or improving reactive oxygen disorder, comprising glutathione trisulfide or a pharmacologically acceptable salt thereof.

### Advantageous Effects of Invention

According to the present invention, it is possible to provide a composition for preventing or improving hepatic dysfunction. The composition for prevention or improvement according to the present invention comprises glutathione trisulfide or a pharmacologically acceptable salt thereof, thereby being capable of protecting the liver through a high anti-inflammatory or anti-oxidant action in vivo, and preventing or improving hepatic dysfunction. In addition, the composition according to the present invention can be administered either short-term or long-term, and it can be routinely ingested particularly in a case of a health functional food or a food composition according to the present invention, which is ingested with a focus on the specific function of the invention.

### Brief Description of Drawings

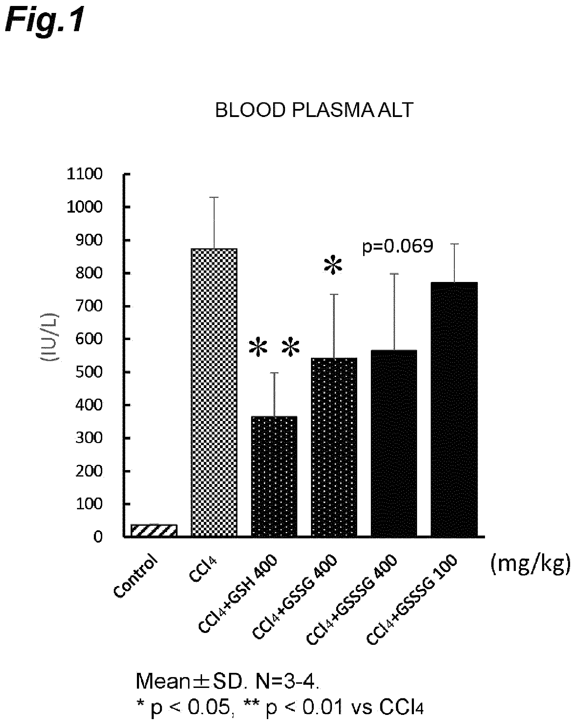

FIG. 1 is a graph showing blood plasma aspartate aminotransferase (AST) levels in a model in which mice are subjected to an once-daily oral administration of each of GSH, GSSG, or GSSSG for a total of three consecutive days and then hepatic dysfunction is induced by carbon tetrachloride (CCl₄). Values are indicated in terms of mean ± SD. Significant differences as compared with a CCl₄ group are shown as ** at p < 0.01 and * at p < 0.05.

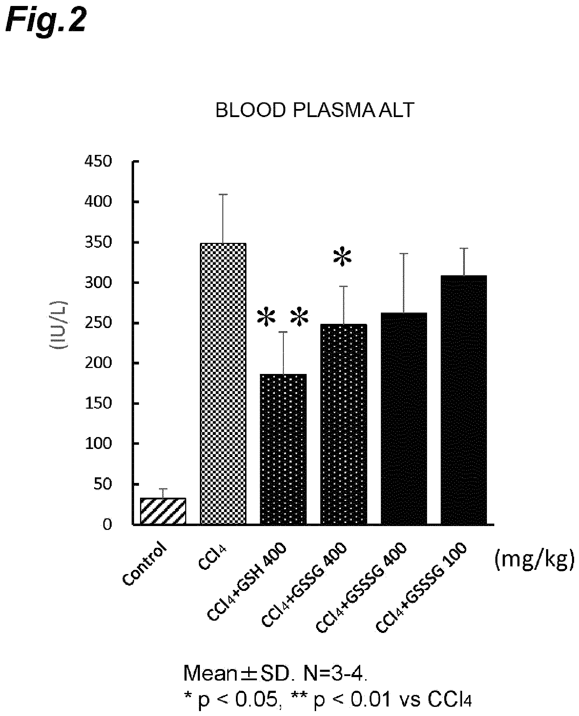

FIG. 2 is a graph showing blood plasma alanine aminotransferase (ALT) levels in a model in which mice are subjected to a once-daily oral administration of each of GSH, GSSG, or GSSSG for a total of three consecutive days and then hepatic dysfunction is induced by carbon tetrachloride (CCl₄). Values are indicated in terms of mean ± SD. Significant differences as compared with a CCl₄ group are shown as ** at p < 0.01 and * at p < 0.05.

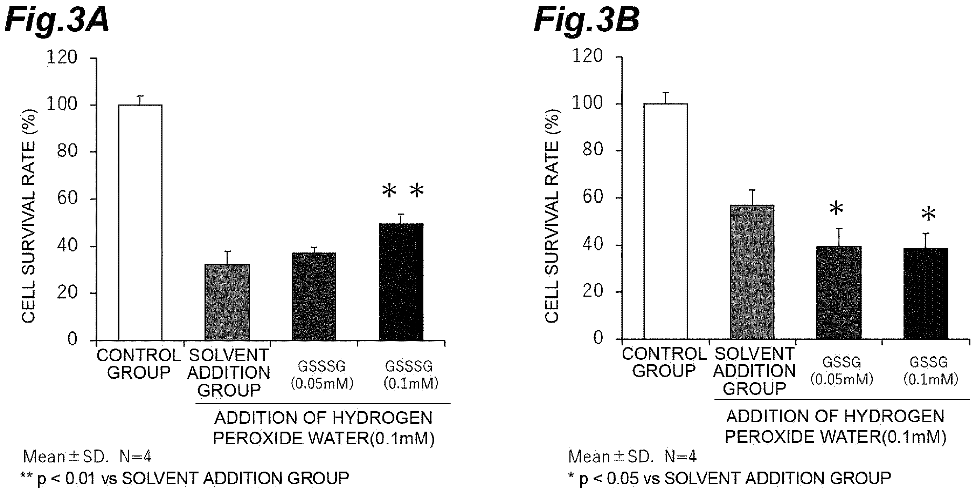

FIG. 3 shows graphs showing effects of GSSSG or GSSG on hepatic dysfunction due to hydrogen peroxide water. FIG. 3(a) is a graph showing the cell survival rate in a case where GSSSG has been added. FIG. 3(b) is a graph showing the cell survival rate in a case where GSSG has been added.

### Description of Embodiments

Hereinafter, the contents of the present invention will be described in detail; however, the present invention is not limited to the following embodiments. Hereinafter, glutathione trisulfide is also referred to as GSSSG.

The composition for prevention or improvement and the method for prevention or improvement according to the present invention may be administered to or applied to humans.

Hepatic dysfunction in the present invention refers to a condition in which abnormal liver function is observed. Specific examples thereof include a condition in which hepatocytes are ruptured, and thus deviation enzymes are leaked into the blood, a condition in which a lot of triglycerides are accumulated in the liver tissue as compared with a normal liver, and a condition in which hepatic parenchymal cells have undergone degeneration or necrosis.

The hepatic dysfunction in the present invention may be a condition that is accompanied with a hepatic function disease, where abnormal liver function is observed, or it may be a condition that is not accompanied with a hepatic function disease, where abnormal liver function is observed. The hepatic dysfunction in the present invention may be hepatic dysfunction due to reactive oxygen disorder. The reactive oxygen disorder refers to a disorder induced by reactive oxygen species. Examples of the hepatic function disease include hepatitis B, hepatitis C, alcoholic liver disorder, nonalcoholic fatty liver disease, drug-induced liver disorder, and chronic hepatitis.

The present invention may be a composition for preventing or improving reactive oxygen disorder, which comprises glutathione trisulfide or a pharmacologically acceptable salt thereof. GSSSG that is an active ingredient according to the present invention has an excellent effect of preventing or improving reactive oxygen disorder. Therefore, the range of application of GSSSG is not limited to the liver, and GSSSG can also be used for diseases, symptoms, or the like in other tissues, which are associated with reactive oxygen disorder.

Examples of the indicator for hepatic dysfunction include blood concentrations (for example, serum concentrations) of aspartate aminotransferase (AST), alanine aminotransferase (ALT), and the like. In a case where a measured value of any one or more of these indicators exceeds a normal value (reference value) range, a patient can be determined to have hepatic dysfunction. Normal values of both blood AST and blood ALT can be 30 IU/L or less based on the determination classification table (revised on April 1, 2020, available from the Japanese Society of Ningen Dock) for various examination items presented by the Public Interest Incorporated Association, Japanese Society of Ningen Dock.

The composition according to the present invention can also prevent or improve symptoms related to hepatic dysfunction. That is, the present invention can also be referred to as a composition for preventing or improving symptoms related to hepatic dysfunction. Examples of the symptoms related to hepatic dysfunction include subjective symptoms such as fatigue, sense of fatigue, malaise, nausea, anorexia, gastrointestinal symptoms, jaundice, rough skin, swelling, and fever, which are caused by decreased liver function, as well as hepatic cirrhosis, liver cancer, and complications thereof.

In the present invention, examples of the pharmacologically acceptable salt include salts with inorganic acids such as hydrochloric acid, hydrobromic acid, sulfuric acid, nitric acid, and phosphoric acid; salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid; salts with alkali metals such as sodium and potassium; salts with alkaline earth metals such as calcium and magnesium; ammonium salts; and salts with amino acids such as arginine.

In the present invention, GSSSG or a pharmacologically acceptable salt thereof may have crystal polymorphism, but is not limited to any crystal polymorphism, and it may be a single product of any crystalline form or may be a mixture. In addition, in the present invention, GSSSG or a pharmacologically acceptable salt thereof also includes an amorphous substance. In addition, in the present invention, GSSSG or a pharmacologically acceptable salt thereof includes an anhydride and a solvate (particularly a hydrate).

The composition for prevention or improvement according to the present invention can be provided as a food composition for preventing or improving hepatic dysfunction and symptoms related thereto. In addition, the composition for prevention or improvement according to the present invention can also be provided as a pharmaceutical composition for preventing or improving hepatic dysfunction and symptoms related thereto.

The food composition includes, in addition to a general food, a food with a function display, and a food for specified health uses. The form of the food composition can be a form that is suitable for edible use, for example, a solid shape, a liquid shape, a granular shape, a powder shape, a capsule shape, a cream shape, or a paste shape.

In a case where the composition for preventing or improving hepatic dysfunction according to the present invention is provided as a pharmaceutical composition, it can be mixed with a pharmacologically acceptable additive and formulated in a formulation form suitable for application to the affected part. The composition for preventing or improving hepatic dysfunction according to the present invention, which is a pharmaceutical composition, can be orally administered, for example, as a tablet, a capsule agent, a powdered drug, a granule, a liquid agent, or a syrup, or can also be parenterally administered as an injection agent, a transfusion, or a suppository. Oral administration is preferred since the administration can be simply carried out. These dosage forms can be formulated according to the publicly known formulation techniques. In a case of a solid agent, it is possible to blend pharmaceutically acceptable excipients, for example, starch, lactose, purified white sugar, glucose, crystalline cellulose, carboxycellulose, carboxymethyl cellulose, carboxyethyl cellulose, calcium phosphate, magnesium stearate, and gum arabic, and it is possible to blend, as necessary, a lubricant, a binder, a disintegrating agent, a coating agent, a coloring agent, and the like. In addition, in a case of a liquid agent, it is possible to blend a stabilizer, a dissolution aid, a suspending agent, an emulsifying agent, a buffering agent, a preservative, and the like.

The dose of the composition for preventing or improving hepatic dysfunction according to the present invention varies depending on the symptom, age, administration method, dose form, and the like; however, in a typical case, 0.5 to 2,000 mg, preferably 10 to 1,000 mg, and most preferably 100 to 800 mg per day can be administered to an adult as the above-described compound, and it is preferable to administer 1 to 800 mg, once a day or several times a day. In a case where the dose of GSSSG is in these ranges, it is considered that the prevention effect or improvement effect on hepatic dysfunction and symptoms related thereto is increased higher.

### [Examples]

Hereinafter, a test example in which the effect of glutathione trisulfide on hepatic dysfunction has been investigated is shown. The present invention is not limited by the test example. CCl₄ means carbon tetrachloride. Purified water was used as vehicle.

GSSSG dihydrate prepared by the method described in Patent Literature 1 was crushed in a mortar. To 10 mL of distilled water, the crushed GSSSG dihydrate (200 mg in terms of anhydride) was added and suspended. Then, twice as much sodium hydrogen carbonate as the GSSSG anhydride was added thereto on a molar basis, and the resultant mixture was mixed for 10 minutes. The obtained GSSSG solution was further diluted with distilled water to obtain administration samples 3 and 4. In addition, reduced glutathione (GSH) or oxidized glutathione (GSSG), manufactured by KYOWA HAKKO BIO Co., Ltd., was dissolved in distilled water to obtain each of administration samples 1 and 2.

Four-week-old male ICR mice (Charles River Laboratories Japan, Inc.) were acclimated for 1 week and then subjected to a test at 5 weeks of age. During the test period, the mice were allowed to freely drink water regarding water, allowed to freely ingest a solid feed CE-2 (CLEA Japan, Inc.), and kept at a temperature of 20°C to 24°C, a humidity of 50% to 60%, and for 12 hours of light-dark cycle. Thereafter, the mice were divided into groups 1 to 6 so that each group included 3 or 4 mice.
Group 1: Normal (vehicle - no symptoms induced)
Group 2: Vehicle + CCl₄
Group 3: GSH 400 mg/kg/day (administration sample 1) + CCl₄
Group 4: GSSG 400 mg/kg/day (administration sample 2) + CCl₄
Group 5: GSSSG 100 mg/kg/day (administration sample 3) + CCl₄
Group 6: GSSSG 400 mg/kg/day (administration sample 4) + CCl₄

It is noted that the GSSSG doses in the group 5 and the group 6 are approximately 500 mg and 2000 mg, respectively, in terms of the dose per one person having a body weight of 60 kg. The dosage per one person can be estimated by using a human equivalent dose (HED).

After the group dividing, the vehicle, the administration sample 1, the administration sample 2, the administration sample 3, or the administration sample 4 was subjected to an once-daily forced oral administration at the above-described dose for a total of three consecutive days. After 30 minutes from the final oral administration, CCl₄ (0.04 mL/kg) suspended in olive oil was intraperitoneally administered to induce hepatic dysfunction. After 16 hours from the CCl₄ administration, whole blood was collected from the abdominal inferior vena cava under isoflurane inhalation anesthesia, and the blood plasma AST and ALT levels (also denoted as "blood plasma AST level" and "blood plasma ALT level" respectively) were analyzed using a biochemical automated analysis device: FUJI DRI-CHEM 4000 (manufactured by FUJIFILM Corporation)

As shown in FIG. 1 and FIG. 2, the blood plasma AST level and the blood plasma ALT level were increased in the group 2 (CCl₄ + vehicle), and noticeable hepatic dysfunction was observed. On the other hand, in the group 3 (GSH) and the group 4 (GSSG), the significant suppression of the blood plasma AST level and the blood plasma ALT level was observed as compared with the group 2. In addition, a tendency of the suppression of the blood plasma AST level (p = 0.069) and the blood plasma ALT level was observed even in the group 6 (GSSSG:400 mg). From this fact, it has been revealed for the first time that the degree of the hepatic dysfunction is alleviated by the administration of GSSSG when the hepatic dysfunction occurs, and it has been shown that GSSSG can be an excellent composition for preventing or improving hepatic dysfunction. In a case of being administered in vivo, CCl₄ is known to generate trichloromethyl radicals, which induces very severe hepatic lipid peroxidation to cause an acute liver damage. In addition, it is also known that further, various radicals are generated secondarily, and that further, an inflammatory reaction is induced in the living body. It has been suggested that GSSSG can suppress or improve such damages that are caused by CCl₄.

Hereinafter, a test example in which the protective effects of GSSSG and GSSG against hepatocellular damage due to oxidative stress have been investigated is shown. The present invention is not limited by the test example.

A human hepatoma-derived cell line (HepG2: HB-8065^{™}, Lot number: 70029112) was obtained from American Type Culture Collection, and Dulbecco's Modified Eagle Medium containing 10% FBS (Thermo Fisher Scientific, Inc.) was used as a basal medium. Cells were cultured in an incubator of 37°C and 5% CO₂, and the cells that had been passaged to 16 to 25 generations were subjected to experimentation. The used GSSG and GSSSG were those manufactured by KYOWA HAKKO BIO Co., Ltd.

0.15 mL of a cell suspension obtained by adjusting the initial cell concentration to 2 x 10⁴ cells/mL was seeded in a 96-well plate (Thermo Fisher Scientific, Inc.).

After the culturing for 4 hours from the seeding, GSSSG (for the addition group of the final concentration of 0.05 mM and the addition group of the final concentration of 0.1 mM) was added to the culture medium. In addition, apart from this, a group in which only the solvent of GSSSG (PBS in which sodium hydrogen carbonate was dissolved so that the concentration thereof was 15.8 mM) was added was defined as a solvent addition group. After the culturing for 20 hours from the sample addition, the culture medium was replaced with a culture medium (not containing GSSSG) obtained by adding hydrogen peroxide solution (FUJIFILM Wako Pure Chemical Corporation) to the above-described sample addition group and the solvent addition group so that the final concentration thereof was 0.1 mM, and then hepatocellular damage was induced. The control group was the group in which the same amount of PBS was added to the solvent addition group instead of hydrogen peroxide solution. After the culturing for 2 hours from the addition of hydrogen peroxide solution or PBS, the cell survival rate was measured according to the MTT assay, and the degree of hepatocellular damage was evaluated. For GSSG as well, an experiment was carried out in the same manner as in the GSSSG, except that PBS was used as a solvent. The results are shown in FIG. 3 and Table 1.

**[Table 1]**

| | GSSSG | GSSG | | |
|---|---|---|---|---|
| | Cell survival rate (%) | SD | Cell survival rate (%) | SD |
| Control group | 100.0 | 3.7 | 100.0 | 4.6 |
| Solution addition group | 32.5 | 5.5 | 56.8 | 6.6 |
| Sample addition group (0.05 mM) | 36.9 | 2.8 | 39.4 | 7.7 |
| Sample addition group (0.1 mM) | 49.7 | 4.0 | 38.4 | 6.4 |

As shown in FIG. 3 and Table 1, the addition of hydrogen peroxide solution reduced the cell survival rate of HepG2 cells, whereas the group to which GSSSG had been added showed an improved survival rate as compared with the solvent addition group. On the other hand, the cell survival rate in the group to which GSSG had been added was significantly decreased as compared with the solvent addition group. As a result, it has been suggested that GSSSG has an excellent effect of suppressing or improving the damage to the liver due to oxidative stress.

## Claims

1. A composition for preventing or improving hepatic dysfunction, comprising glutathione trisulfide or a pharmacologically acceptable salt thereof.

2. The composition according to claim 1, wherein the hepatic dysfunction is a condition that is not accompanied with a hepatic function disease, where abnormal liver function is observed, or a condition that is accompanied with a hepatic function disease, where abnormal liver function is observed.

3. The composition according to claim 2, wherein the hepatic function disease is at least one selected from the group consisting of hepatitis B, hepatitis C, alcoholic liver disorder, nonalcoholic fatty liver disease, and chronic hepatitis.

4. The composition according to claim 1, wherein the hepatic dysfunction is hepatic dysfunction due to reactive oxygen disorder.

5. The composition according to any one of claims 1 to 4, wherein the composition is a food composition.

6. The composition according to any one of claims 1 to 4, wherein the composition is a pharmaceutical composition.
